# EUROPÄISCHE PATENTSCHRIFT

(11) **EP 2 922 815 B1**
(45) Veröffentlichungstag und Bekanntmachung des Hinweises auf die Patenterteilung: **18.01.2017**
(21) Anmeldenummer: 13795269.3
(22) Anmeldetag: 26.11.2013
(51) Int. Cl.: C07C 67/36, C07C 69/06

(54) **VERFAHREN ZUR HERSTELLUNG VON METHYLFORMIAT DURCH UMSETZUNG VON METHANOL MIT KOHLENMONOXID IN GEGENWART EINES KATALYSATORSYSTEMS, DAS ALKALIFORMIAT UND ALKALIALKOHOLAT ENTHÄLT**
METHOD FOR PRODUCING METHYL FORMATE BY REACTING METHANOL WITH CARBON MONOXIDE IN THE PRESENCE OF A CATALYST SYSTEM WHICH COMPRISES ALKALI FORMATE AND ALKALI ALCOHOLATE
PROCÉDÉ DE PRODUCTION DE MÉTHYLFORMIATE PAR RÉACTION DU MÉTHANOL AVEC DU MONOXYDE DE CARBONE EN PRÉSENCE D'UN SYSTÈME CATALYSEUR RENFERMANT UN FORMIATE ALCALIN ET UN ALCOOLATE ALCALIN

(30) Priorität: 26.11.2012 EP 12194185
(43) Veröffentlichungstag der Anmeldung: 30.09.2015
(73) Patentinhaber: BASF SE, 67056 Ludwigshafen am Rhein (DE)
(72) Erfinder: SCHNEIDER, Daniel, 67227 Frankenthal (DE); MOHL, Klaus-Dieter, 68766 Hockenheim (DE); SCHÄFER, Martin, 67269 Grünstadt (DE); PASCHOLD, Jürgen, 67691 Hochspeyer (DE); TELES, Joaquim Henrique, 67165 Waldsee (DE); RITTINGER, Stefan, 68199 Mannheim (DE)
(86) Internationale Anmeldenummer: PCT/EP2013/074663
(87) Internationale Veröffentlichungsnummer: WO 2014/080026

(56) Entgegenhaltungen:
- WO-A1-01/07392

## Beschreibung

Die vorliegende Erfindung betrifft ein Verfahren zur Herstellung von Methylformiat durch Umsetzung von Methanol mit Kohlenmonoxid in Gegenwart eines Katalysatorsystems, das ein Alkaliformiat und ein Alkalialkoholat enthält.

Methylformiat (Ameisensäuremethylester) ist ein wichtiges Zwischenprodukt zur Herstellung von Ameisensäure, wobei Methylformiat mit Wasser zu Ameisensäure und Methanol hydrolysiert wird. Methylformiat wird darüber hinaus zur Herstellung von Acetaldehyd durch Hydroisomerisierung an Rhodium- oder IridiumKatalysatoren eingesetzt. Darüber hinaus ist die Isomerisierung von Methylformiat zu Essigsäure, sowie die oxidative Umsetzung von Methylformiat mit Methanol an Selen-Katalysatoren zu Dimethylcarbonat beschrieben (Hans-Jürgen Arpe, Industrielle Organische Chemie, 6. Auflage, 2007, Seite 48).

Methylformiat wird seit über 80 Jahren großtechnisch durch Carbonylierung von Methanol hergestellt. Die Carbonylierung erfolgt in der Regel basenkatalysiert, wobei insbesondere Natriummethanolat (Natriummethylat) als Katalysator eingesetzt wird. Die Umsetzung erfolgt in der Regel bei etwa 70°C und bei einem Druck von bis zu 200 bar (Ullmanns Encyclopedia of Technical Chemistry, 6. Auflage (2003), Band 15, Seiten 5 bis 8; Wiley-VCH-Verlag, DOI: 10.1002/14356007.a12_013).

Bei der Carbonylierung von Methanol handelt es sich um eine homogenkatalysierte Gleichgewichtsreaktion, bei der mit zunehmendem Kohlenmonoxid-Partialdruck und sinkender Temperatur das Gleichgewicht in Richtung Methylformiat verschoben wird. Bei der kontinuierlichen Carbonylierung von Methanol ist für ein wirtschaftliches Verfahren mit einer akzeptablen Raumzeitausbeute (RZA) neben der Gleichgewichtslage auch eine ausreichend hohe Reaktionsgeschwindigkeit notwendig. Die Reaktionsgeschwindigkeit der Carbonylierung von Methanol lässt sich durch Erhöhung der Temperatur oder durch Erhöhung des Kohlenmonoxid-Partialdrucks steigern. Eine Erhöhung der Reaktionsgeschwindigkeit durch die vorstehend beschriebenen Methoden ist jedoch mit Nachteilen verbunden. So führt die Erhöhung der Temperatur wie vorstehend beschrieben zu einer Verschlechterung der Gleichgewichtslage, was wiederum eine Verschlechterung der Raumzeitausbeute mit sich bringt. Mittlerweile existieren zahlreiche Verfahren mit unterschiedlichen Ausführungsformen, die zur Erreichung einer akzeptablen Raumzeitausbeute unter hohem Druck (bis zu 200 bar) durchgeführt werden. Für diese Hochdruckverfahren sind jedoch speziell ausgelegte Reaktoren erforderlich, die mit hohen Investitionskosten für den Reaktor verbunden sind.

WO 2001/07392 beschreibt ein Verfahren, zur Herstellung von Methylformiat, bei dem die Umsetzung von Methanol mit Kohlenmonoxid bei einem Kohlenmonoxiddruck von 9 bis 18 MPa (90 bis 180 bar) in Gegenwart von 0,05 bis 0,5 Gew.-% eines Alkalimethanolats, bezogen auf das Gewicht des flüssigen Reaktorfeeds, erfolgt. In der Beschreibung von WO 2001/07392 wird darauf hingewiesen, dass das als Katalysator eingesetzte Alkalimethanolat insbesondere durch zwei unerwünschte aber unvermeidliche Nebenreaktionen zu dem katalytisch unwirksamen Alkaliformiat umgesetzt wird. Das Alkaliformiat wird auch als verbrauchter Katalysator bzw. Katalysatorabbauprodukt bezeichnet. Das Alkaliformiat kann dabei gemäß Gleichung (i) durch Reaktion von Alkalimethanolat mit Methylformiat zu Alkaliformiat und Dimethylether gebildet werden. Darüber hinaus entsteht Alkaliformiat in Gegenwart von Wasserspuren gemäß Gleichung (ii) durch Hydrolyse aus Alkalimethanolat und Methylformiat, wobei Methanol und Alkaliformiat entstehen. Die Nebenreaktionen (i) und (ii) werden nachfolgend am Beispiel der Bildung von Natriumformiat aus Natriummethanolat verdeutlicht.

(i) NaOCH₃ + HCOOCH₃ → HCOONa + CH₃OCH₃

(ii) NaOCH₃ + H₂O + HCOOCH₃ → HCOONa + 2 CH₃OH

Bei dem Verfahren gemäß WO 2001/07392 wird aus dem Reaktoraustrag das gebildete Methylformiat in einer Destillationsvorrichtung ausgetrieben. Das als Katalysator eingesetzte unverbrauchte Alkalimethanolat kann zum Carbonylierungsreaktor rückgeführt werden. Hierbei ist es jedoch erforderlich die Katalysatorabbauprodukte vor der Rückführung in einer Entsalzungsvorrichtung zu entfernen, um die Abscheidung von Salzen zu verhindern. Das als Katalysatorabbauprodukt auftretende Alkaliformiat kann aufgrund seiner unzureichenden Löslichkeit zu Ablagerungen in den Apparaten und Rohrleitungen bis hin zur Verstopfung von Rohren und Ventilen führen. Nach der Lehre von WO 2001/07392 liegt der Gehalt an Alkaliformiat am Reaktoraustritt vorzugsweise im Bereich von 0,1 bis 0,3 Gew.-%.

WO 2003/089398 beschreibt ebenfalls ein Verfahren zur Herstellung von Methylformiat aus Methanol und Kohlenmonoxid in Gegenwart eines Alkalialkoholats, in Konzentrationen von 0,01 bis 2 Mol pro kg flüssiges Reaktionsgemisch. Auch dort wird das Alkaliformiat als unerwünschtes Katalysatorabbauprodukt beschrieben, das zur Verhinderung von salzartigen Ablagerungen ausgeschleust wird. Bei diesem Verfahren ist zudem eine apparativ aufwändige Rückführung eines Gasstroms erforderlich, der eine mittlere Gasleerrohrgeschwindigkeit im Bereich 1 bis 20 m/s aufweist.

Auch Ullmanns Encyclopedia of Technical Chemistry (2005, Kapitel "Formic Acid", Seiten 6 bis 7; Wiley-VCH-Verlag, DOI: 10.1002/14356007.a12_013) beschreibt, dass sich aus Natriummethanolat in einer unerwünschten Nebenreaktion mit Methylformiat Dimethylether und das katalytisch inaktive Natriumformiat bildet.

Im PEP-Report (Process Economics Program "Formic Acid", 1983, Seiten 50 bis 52) wird ein Verfahren zur Herstellung von Methylformiat aus Methanol und Kohlenmonoxid in Gegenwart von Natriumformiat beschrieben. Das gebildete Alkaliformiat wird als verbrauchter oder inaktivierter Katalysator bezeichnet. Der Katalysator, Natriummethanolat, kann zum Reaktor rückgeführt werden. Hierzu ist es jedoch notwendig, das gebildete Natriumformiat aus dem Verfahren in solchen Mengen auszuschleusen, dass das molare Verhältnis von verbrauchtem Katalysator (Natriumformiat) zu Katalysator (Natriummethanolat) maximal äquimolar ist.

US 2004/0171704 beschreibt ein Verfahren zur Herstellung von Methanol oder Ameisensäureestern durch Umsetzung von Kohlenmonoxid mit einem Alkohol. Als Katalysatoren werden bevorzugt Alkalimetallsalze eingesetzt. Ziel der US 2004/0171704 ist es die Umsetzung von Kohlenmonoxid mit dem Alkohol auch in Gegenwart von Wasser und/oder Kohlendioxid zu ermöglichen. Als Katalysatoren werden Alkalicarbonate, Alkalinitrate, Alkaliphosphate, Alkaliacetate und Alkaliformiate beschrieben. Der Einsatz von Alkalialkoholaten wird ausdrücklich ausgeschlossen, da diese in Gegenwart von Wasser und/oder Kohlendioxid inaktiviert werden.

EP 0 596 483 beschreibt ein Verfahren zur Herstellung von Methylformiat durch Carbonylierung von Methanol in Gegenwart von Natrium- oder Kaliummethanolat als Katalysator. Auch EP 0 596 483 beschreibt, dass sich das als Katalysator eingesetzte Alkalimethanolat (Alkalimethylat) zu inaktiven Zersetzungsprodukten, wie Natrium- oder Kaliumformiat, Natrium- oder Kaliumcarbonat und Natrium- oder Kaliumhydrogencarbonat umwandelt. Die Zersetzungsprodukte werden dabei periodisch über einen Filter entfernt, wobei am Reaktorausgang ca. 0,9 Gew.-% Zersetzungsprodukte anfallen. Die Zersetzungsprodukte setzen sich dabei aus ca. 38 Gew.-% Natriumformiat, 42 Gew.-% Natriumhydrogencarbonat, 15 Gew.-% Natriumcarbonat und 6 Gew.-% Natriummethanolat zusammen. Um den Katalysatorverbrauch zu verringern und die Raumzeitausbeute zu steigern, wird die Reaktion in Gegenwart eines speziellen Natrium- oder Kalium-oxaperfluoralkansulfonats und einer starken organischen Base durchgeführt.

Nachteilig an den im Stand der Technik beschriebenen Verfahren ist, das zur Erreichung akzeptabler Raumzeitausbeuten sehr hohe Drücke erforderlich sind und/oder apparativ aufwändige Rückführungen von Kreisgasströmen mit hohen Gasleerrohrgeschwindigkeiten erforderlich sind. Diese Verfahren erfordern speziell ausgelegte Reaktoren, die mit hohen Investitionskosten verbunden sind. Bei dem in EP 0 596 483 beschriebenen Verfahren sind zwar niedrigere Kohlenmonoxid-Partialdrücke von 3,0 MPa (30 bar) möglich, es ist jedoch der Einsatz von sehr teuren Natrium- oder Kalium-oxa-perfluoralkansulfonaten und starken organischen Basen erforderlich.

Aufgabe der vorliegenden Erfindung war es ein Verfahren bereitzustellen, das Methylformiat in guten Raumzeitausbeuten liefert. Ferner soll das Verfahren eine einfachere Verfahrensführung erlauben, als die im Stand der Technik beschriebenen Verfahren, insbesondere ohne die im Stand der Technik beschriebenen aufwändigen Hochdruckreaktoren und ohne teure Katalysatorzusätze, wie beispielsweise Kalium-oxa-perfluoralkansulfonate und starke organische Basen.

Diese Aufgabe wird gelöst durch ein Verfahren zur Herstellung von Methylformiat durch Carbonylierung von Methanol mit Kohlenmonoxid in einem Carbonylierungsreaktor in Gegenwart eines Katalysatorsystems, das Alkaliformiat und Alkalialkoholat enthält, unter Erhalt eines Reaktionsgemischs (R_{G}), das Methylformiat, Alkaliformiat, Alkalialkoholat sowie gegebenenfalls nicht umgesetztes Methanol und nicht umgesetztes Kohlenmonoxid enthält, und aus dem Carbonylierungsreaktor entnommen wird, wobei das Reaktionsgemisch (R_{G}) mindestens 0,5 Gew.-% Alkalialkoholat bezogen auf das Gesamtgewicht des Reaktionsgemischs (R_{G}) enthält und das molare Verhältnis von Alkaliformiat zu Alkalialkoholat im Reaktionsgemisch (R_{G}) größer als 1 ist.

Bei dem erfindungsgemäßen Verfahren zur Herstellung von Methylformiat aus Methanol und Kohlenmonoxid werden sehr gute Raumzeitausbeuten erzielt, die teilweise sogar über den im Stand der Technik beschriebenen Raumzeitausbeuten liegen. Das erfindungsgemäße Verfahren hat den Vorteil, dass es auch bei niedrigeren Drücken Methylformiat in guten Ausbeuten zugänglich macht. Hierdurch können bei der Reaktorauslegung Kosten eingespart werden. Darüber hinaus liefert das erfindungsgemäße Verfahren sehr gute Raumzeitausbeuten, ohne dass der Einsatz teurer Additive wie Kalium-oxa-perfluoralkansulfonate und starker organischer Basen erforderlich wird. Auch die im Stand der Technik beschriebenen Probleme von Salzablagerungen, die zu Verstopfungen von Rohren und Ventilen führen können, lassen sich durch das erfindungsgemäße Verfahren vermindern oder sogar vollständig verhindern.

Im Stand der Technik bestand das Vorurteil, dass nur Alkalialkoholate im Hinblick auf die Carbonylierung von Methanol katalytisch aktiv sind. Alkaliformiate werden im Stand der Technik hingegen als katalytisch inaktiv beschrieben. Alkaliformiate werden im Stand der Technik auch als verbrauchte Katalysatoren beschrieben, die aus dem Reaktionsgemisch entfernt werden müssen und durch neues, katalytisch aktives Alkalialkoholat ersetzt werden müssen.

Es wurde überraschenderweise festgestellt, dass Alkaliformiat in Kombination mit Alkalialkoholat, entgegen dem in Stand der Technik bestehenden Vorurteil, im Hinblick auf die Carbonylierung von Methanol katalytisch aktiv ist. Dies gilt insbesondere für eine Mischung, die Alkaliformiat und Alkalialkoholat enthält in der das molare Verhältnis von Alkaliformiat zu Alkalialkoholat größer 1, bevorzugt größer 2, besonders bevorzugt größer 3 und insbesondere größer 5 ist.

Gegenstand der vorliegenden Erfindung ist daher auch die Verwendung einer Mischung, die ein Alkaliformiat und ein Alkalialkoholat enthält und in der das molare Verhältnis von Alkaliformiat zu Alkalialkoholat größer 1, bevorzugt größer 2, besonders bevorzugt größer 3 und insbesondere größer 5 ist, als Katalysatorsystem zur Herstellung von Methylformiat durch Carbonylierung von Methanol mit Kohlenmonoxid.

### Umsetzung von Methanol mit Kohlenmonoxid zu Methylformiat

Das im erfindungsgemäße Verfahren eingesetzt Kohlenmonoxid kann fest, flüssig oder gasförmig eingesetzt werden. Kohlenmonoxid kann als Reinstoff, das heißt mit einem Gehalt von mindestens 95 Gew.-%, bevorzugt mindestens 97 Gew.-% und besonders bevorzugt mindestens 99 Gew.-% eingesetzt werden. Bevorzugt wird Kohlenmonoxid gasförmig eingesetzt. Das eingesetzte Kohlenmonoxid ist bevorzugt weitestgehend frei von Kohlendioxid, d.h. in der Regel weniger als 1 Gew.-% Kohlendioxid, bevorzugt weniger als 0,5 Gew.-% Kohlendioxid, jeweils bezogen auf das Gesamtgewicht des Kohlenmonoxid-haltigen Gasgemischs. Es ist auch möglich, Kohlenmonoxid enthaltende Gasgemische einzusetzen, die neben Kohlenmonoxid weitere Inerte wie beispielsweise Stickstoff, Wasserstoff, Methan oder Edelgase enthalten. Im Allgemeinen liegt der Gehalt an Inerten jedoch unterhalb von 10 Gew.-% bezogen auf das Gesamtgewicht des Kohlenmonoxid-haltigen Gasgemisches. Größere Mengen sind zwar gegebenenfalls ebenfalls tolerierbar, bedingen aber in der Regel die Anwendung höherer Drücke, wodurch zusätzliche Kompressionsenergie erforderlich wird. Das Kohlenmonoxid entstammt in der Regel aus dem Fachmann geläufigen Kohlenmonoxid-Quellen, wie beispielsweise Synthesegas.

Das im erfindungsgemäßen Verfahren eingesetzte Methanol ist in einer bevorzugten Ausführungsform im Wesentlichen wasserfrei, d.h. das eingesetzte Methanol enthält maximal 250 Gew.-ppm, bevorzugt maximal 100 Gew.-ppm und besonders bevorzugt maximal 50 Gew.-ppm Wasser, jeweils bezogen auf das Gesamtgewicht aus eingesetztem Methanol und dem darin enthaltenen Wasser.

In einer bevorzugten Ausführungsform wird die Carbonylierung von Methanol mit Kohlenmonoxid wasserfrei durchgeführt. Unter wasserfrei wird dabei verstanden, dass das Reaktionsgemisch (R_{G}) maximal 250 Gew.-ppm, bevorzugt maximal 100 Gew.-ppm und besonders bevorzugt maximal 50 Gew.-ppm Wasser enthält, jeweils bezogen auf das Gesamtgewicht des Reaktionsgemischs (R_{G}).

Das im erfindungsgemäßen Verfahren eingesetzte Alkalialkoholat kann als Feststoff oder als Lösung in einem geeigneten Lösungsmittel eingesetzt werden. Es können Mischungen aus zwei oder mehreren Alkalialkoholaten eingesetzt werden. Unter Alkalialkoholat werden im Rahmen der vorliegenden Erfindung sowohl ein Alkalialkoholat, als auch Mischungen aus zwei oder mehr Alkalialkoholaten verstanden. Bevorzugt wird jedoch nur ein Alkalialkoholat eingesetzt. In einer bevorzugten Ausführungsform wird ein Alkalimethanolat gelöst in Methanol eingesetzt.

Das im erfindungsgemäßen Verfahren eingesetzte Alkaliformiat kann ebenfalls als Feststoff oder als Lösung in einem geeigneten Lösungsmittel eingesetzt werden. Es können Mischungen aus zwei oder mehreren Alkaliformiaten eingesetzt werden. Unter Alkaliformiat wird im Rahmen der vorliegenden Erfindung sowohl ein Alkaliformiat, als auch Mischungen aus zwei oder mehr Alkaliformiaten verstanden. Bevorzugt wird jedoch nur ein Alkaliformiat eingesetzt. In einer bevorzugten Ausführungsform wird ein Alkaliformiat gelöst in Methanol eingesetzt. Das Alkaliformiat kann dem Carbonylierungsreaktor auch durch Rückführung aus nachgelagerten Aufarbeitungsstufen zugeführt werden.

Die Alkalimetallkomponenten des Alkaliformiats und des Alkalialkoholats können unabhängig voneinander ausgewählt werden aus der Gruppe bestehend aus Lithium, Natrium, Kalium, Rubidium und Cäsium. Das Alkaliformiat kann daher ausgewählt sein aus der Gruppe bestehend aus Lithiumformiat, Natriumformiat, Kaliumformiat, Rubidiumformiat und Cäsiumformiat. Bevorzugt ist das Alkaliformiat ausgewählt aus der Gruppe bestehend aus Natriumformiat und Kaliumformiat. Besonders bevorzugt ist Kaliumformiat. Das Alkalialkoholat kann ausgewählt sein aus der Gruppe bestehend aus Lithiumalkoholat, Natriumalkoholat, Kaliumalkoholat, Rubidiumalkoholat und Cäsiumalkoholat. Bevorzugt ist das Alkalialkoholat ausgewählt aus der Gruppe bestehend aus Natriumalkoholat und Kaliumalkoholat. Besonders bevorzugt ist Kaliumalkoholat.

Als Alkoholatkomponente des Alkalialkoholats sind Alkoholatanionen mit 1 bis 12 Kohlenstoffatomen geeignet, wie beispielsweise Methanolat, Ethanolat, 1-Propanolat, 2-Propanolat, 1-Butanolat, 2-Butanolat, 2-Methyl-1-propanolat, 2-Methyl-2-propanolat, 1-Pentanolat, Isoamylat, 1-Hexanolat, 1-Heptanolat, 1-Octanolat, 2-Ethyl-1-hexanolat, 1-Nonanolat, 3,5,5-Trimethyl-1-hexanolat, 2,6-Dimethyl-4-heptanolat und 1-Decanolat. Als Alkoholat besonders bevorzugtes ist Methanolat.

Besonders bevorzugt sind die Alkalimetallkomponenten des Alkaliformiats und des Alkalialkoholats identisch.

Insbesondere bevorzugt ist das Alkaliformiat Kaliumformiat und das Alkalialkoholat ist Kaliummethanolat.

Im erfindungsgemäßen Verfahren wird als Katalysatorsystem bevorzugt eine Mischung von Alkaliformiat und Alkalialkoholat eingesetzt, wobei das molare Verhältnis von Alkaliformiat zu Alkalialkoholat in der Mischung größer als 1 ist. Bevorzugt liegt das molare Verhältnis von Alkaliformiat zu Alkalialkoholat im Bereich von 2 bis 20, besonders bevorzugt im Bereich von 2 bis 15, ganz besonders bevorzugt im Bereich von 3 bis 10 und insbesondere im Bereich von 3 bis 8, wobei als Alkaliformiat Kaliumformiat und als Alkalialkoholat Kaliummethanolat besonders bevorzugt sind.

Bevorzugt ist das molare Verhältnis von Alkaliformiat zu Alkalialkoholat im Reaktionsgemisch (R_{G}) größer 2, besonders bevorzugt größer 3 und insbesondere bevorzugt größer 5.

Bevorzugt liegt das molare Verhältnis von Alkaliformiat zu Alkalialkoholat im Reaktionsgemisch (R_{G}) im Bereich von 2 bis 20, besonders bevorzugt im Bereich von 2 bis 15, ganz besonders bevorzugt im Bereich von 3 bis 10 und insbesondere im Bereich von 3 bis 8, wobei als Alkaliformiat Kaliumformiat und als Alkalialkoholat Kaliummethanolat besonders bevorzugt sind.

In einer bevorzugten Ausführungsform wird im erfindungsgemäßen Verfahren ein Katalysatorsystem eingesetzt, das im Wesentlichen aus der vorstehend beschriebenen Mischung aus Alkaliformiat und Alkalialkoholat besteht, wobei eine Mischung, die im Wesentlichen aus Kaliumformiat und Kaliummethanolat bestehen besonders bevorzugt ist.

Das heißt im erfindungsgemäßen Verfahren werden neben der als Katalysatorsystem eingesetzten Mischung aus Alkaliformiat und Alkalialkoholat im Allgemeinen maximal 1 Gew.- %, bevorzugt maximal 0,5 Gew.-% und besonders bevorzugt maximal 0,1 Gew.-% und insbesondere keine weiteren katalytisch wirksamen Substanzen eingesetzt, die die Carbonylierung von Methanol mit Kohlenmonoxid katalysieren, jeweils bezogen auf das Gesamtgewicht der als Katalysatorsystem eingesetzten Mischung und der gegebenenfalls vorhandenen weiteren katalytisch wirksamen Substanzen. In einer bevorzugten Ausführungsform besteht die als Katalysatorsystem eingesetzte Mischung aus Alkaliformiat und Alkalialkoholat, wobei eine Mischung, die aus Kaliumformiat und Kaliummethanolat besteht besonders bevorzugt ist.

Im erfindungsgemäßen Verfahren sind in einer bevorzugten Ausführungsform keine Alkali-oxa-perfluorsulfonate sowie keine starken organischen Basen mit einem pKₐ Wert größer 8,7 vorhanden. Beispiele für Alkali-oxy-perfluorsulfonate, die im erfindungsgemäßen Verfahren nicht zugegen sind, sind solche der allgemeinen Formel

CF₃CF₂(OCFXCF₂)ₚ OCF₂SO₃M

mit p = 0 bis 2, X = F, CF₃ und M = Na, K.

Gegenstand der vorliegenden Erfindung ist daher auch die Verwendung einer Mischung aus einem Alkaliformiat und einem Alkalialkoholat als Katalysatorsystem für die Umsetzung von Methanol mit Kohlenmonoxid zu Methylformiat, wobei das molare Verhältnis von Alkaliformiat und Alkalialkoholat in der Mischung größer als 1 ist. Für die erfindungsgemäße Verwendung der Mischung gelten die vorgenannten Bevorzugungen im Hinblick auf das Alkaliformiat und das Alkalialkoholat sowie die molaren Verhältnisse entsprechend.

Als Carbonylierungsreaktoren können beim erfindungsgemäßen Verfahren prinzipiell alle Reaktoren eingesetzt werden, die für Gas-/Flüssigkeits-Reaktionen geeignet sind. Geeignete Standardreaktoren für gas-flüssig Reaktionssysteme sind beispielsweise in K. D. Henkel, "Reactor Types and Their Industrial Applications", in Ullmann's Encyclopedia of Industrial Chemistry, 2005, Wiley-VCH-Verlag GmbH & CO. KGaA, DOI: 10.1002/14356007.b04_087, Kapitel 3.3 "Reactors for gas-liquid reactions" angegeben. Als Beispiele seien genannt Rührkesselreaktoren, Rohrreaktoren, Strahlschlaufenreaktoren oder Blasensäulen.

Die Carbonylierung von Methanol mit Kohlenmonoxid kann beim erfindungsgemäßen Verfahren kontinuierlich oder diskontinuierlich durchgeführt werden. Bei der diskontinuierlichen Fahrweise wird der Carbonylierungsreaktor mit den gewünschten flüssigen und gegebenenfalls festen Einsatz- und Hilfsstoffen bestückt und anschließend Kohlenmonoxid auf den gewünschten Druck bei der gewünschten Temperatur aufgepresst. Nach Ende der Reaktion wird der Carbonylierungsreaktor im Regelfall entspannt. Bei der kontinuierlichen Fahrweise werden dem Carbonylierungsreaktor Methanol, Kohlenmonoxid, das Katalysatorsystem (Alkaliformiat und Alkalialkoholat, bevorzugt Alkalimethanolat und Alkaliformiat) kontinuierlich zugeführt. Entsprechend wird das Reaktionsgemisch (R_{G}) kontinuierlich aus dem Carbonylierungsreaktor abgeführt, so dass der Flüssigkeitsstand im Carbonylierungsreaktor im Mittel gleich bleibt. Bevorzugt ist die kontinuierliche Carbonylierung von Methanol mit Kohlenmonoxid. Im Carbonylierungsreaktor sind im Allgemeinen eine Flüssigphase und eine Gasphase vorhanden. Die Carbonylierungsreaktion findet im Allgemeinen in der Flüssigphase statt.

Unter Reaktionsgemisch (R_{G}) wird erfindungsgemäß der flüssige Anteil unter den Reaktionsbedingungen der Carbonylierung verstanden, der aus dem Carbonylierungsreaktor entnommen wird.

Das Reaktionsgemisch (R_{G}) beschreibt somit die Zusammensetzung der flüssigen Phase, die aus dem Carbonylierungsreaktor entnommen wird, unter dem Reaktionsdruck der Carbonylierung, d.h. vor der Entspannung.

Die Carbonylierungsreaktion von Methanol mit Kohlenmonoxid erfolgt im Carbonylierungsreaktor im Allgemeinen in der Flüssigphase bei einem Gesamtdruck im Bereich von 30 bis 100 bar, bevorzugt im Bereich von 30 bis 70 bar und besonders bevorzugt im Bereich von 50 bis 65 bar sowie bei einer Temperatur im Bereich von 60 bis 140 °C, bevorzugt im Bereich von 65 bis 110 °C und besonders bevorzugt im Bereich von 70 bis 100 °C. In einer ganz besonders bevorzugten Ausführungsform erfolgt die Carbonylierung im Carbonylierungsreaktor bei einer Temperatur im Bereich von 70 bis 100°C und einem Gesamtdruck im Bereich von 50 bis 65 bar.

Das molare Feedverhältnis der Menge des dem Carbonylierungsreaktor zugeführten Methanols zu der Menge des dem Carbonylierungsreaktor zugeführten Kohlenmonoxids beträgt beim erfindungsgemäßen Verfahren im Allgemeinen von 1 bis 5, bevorzugt von 2 bis 5, besonders bevorzugt von 2,5 bis 4 und insbesondere von 3 bis 4. Die Menge des dem Carbonylierungsreaktor zugeführten Methanols setzt sich zusammen aus dem frisch zugeführten Methanol sowie dem gegebenenfalls aus nachgelagerten Aufarbeitungsstufen rückgeführten Methanol. Die Menge des dem Carbonylierungsreaktor zugeführten Kohlenmonoxids setzt sich zusammen aus dem frisch zugeführten Kohlenmonoxid, sowie dem gegebenenfalls aus nachgelagerten Aufarbeitungsstufen rückgeführten Kohlenmonoxid.

Das molare Feedverhältnis der Menge des dem Carbonylierungsreaktor zugeführten Methanols zu der Menge des dem Carbonylierungsreaktor zugeführten Alkalialkoholats, bevorzugt des Kaliummethanolats, beträgt beim erfindungsgemäßen Verfahren im Allgemeinen von 100 bis 400, bevorzugt von 150 bis 350, besonders bevorzugt von 200 bis 350 und insbesondere von 230 bis 330. Die Menge des dem Carbonylierungsreaktor zugeführten Alkalialkoholats setzt sich zusammen aus dem frisch zugeführten Alkalialkoholat sowie dem gegebenenfalls aus nachgelagerten Aufarbeitungsstufen rückgeführten Alkalialkoholat.

Das molare Feedverhältnis der Menge des dem Carbonylierungsreaktor zugeführten Methanols zu der Menge des dem Carbonylierungsreaktor zugeführten Alkaliformiats, bevorzugt des Kaliumformiats, beträgt beim erfindungsgemäßen Verfahren im Allgemeinen von 25 bis 400, bevorzugt von 30 bis 200, besonders bevorzugt von 30 bis 100 und insbesondere von 30 bis 50. Die Menge des dem Carbonylierungsreaktor zugeführten Alkaliformiats setzt sich zusammen aus dem frisch zugeführten Alkaliformiat sowie dem gegebenenfalls aus nachgelagerten Aufarbeitungsstufen rückgeführten Alkaliformiat.

Unter "frisch zugeführt" im Bezug auf Methanol, Kohlenmonoxid, Alkalialkoholat und Alkaliformiat werden im Rahmen der vorliegenden Erfindung die Komponenten verstanden, die nicht aus nachgelagerten Aufarbeitungsstufen rückgeführt werden. Hierunter sind Komponenten zu verstehen, die nicht dem erfindungsgemäßen Verfahren entstammen, sondern von außen dem erfindungsgemäßen Verfahren zugeführt werden.

Die Zugabe von frisch zugeführtem Alkaliformiat ist jedoch nicht zwingend erforderlich. Alkaliformiat entsteht bei der Carbonylierung von Methanol gemäß den vorstehend beschriebenen Nebenreaktionen (i) und/oder (ii). In einer bevorzugten Ausführungsform des erfindungsgemäßen Verfahrens wird dem Carbonylierungsreaktor kein frisches Alkaliformiat zugeführt und das Alkaliformiat entstammt ausschließlich aus dem aus einer nachgelagerten Aufarbeitungsstufe rückgeführten Alkaliformiat. Bei dieser Ausführungsform wird das Alkaliformiat im erfindungsgemäßen Verfahren so lange aufgepegelt, bis im Reaktionsgemisch (R_{G}) die erfindungsgemäße Konzentration an Alkaliformiat erreicht ist.

Bevorzugt werden Alkalialkoholat und Alkaliformiat dem Reaktor gelöst in Methanol zugeführt.

Beim erfindungsgemäßen Verfahren wird aus dem Carbonylierungsreaktor ein Reaktionsgemisch (R_{G}) entnommen, das Methylformiat, Alkaliformiat, Alkalialkoholat sowie gegebenenfalls nicht umgesetztes Methanol und nicht umgesetztes Kohlenmonoxid enthält. Die Zusammensetzung des Reaktionsgemischs (R_{G}) im Hinblick auf das darin enthaltene Alkaliformiat und Alkalialkoholat richtet sich nach dem eingesetzten Katalysatorsystem. Für die Zusammensetzung des Reaktionsgemischs (R_{G}) im Hinblick auf das darin enthaltene Alkaliformiat und Alkalialkoholat gelten somit die vorstehend aufgeführten Angaben und Bevorzugungen für das Katalysatorsystem entsprechend.

In einer bevorzugten Ausführungsform wird als Katalysatorsystem eine Mischung eingesetzt, die im Wesentlichen aus Kaliumformiat und Kaliummethanolat besteht, wobei ein Reaktionsgemisch (R_{G}) erhalten wird, das im Wesentlichen aus Methylformiat, Kaliumformiat und Kaliummethanolat sowie gegebenenfalls aus nicht umgesetztem Methanol und gegebenenfalls aus nicht umgesetztes Kohlenmonoxid besteht. Unter "im Wesentlichen besteht" wird im Rahmen der vorliegenden Erfindung in Bezug auf das Reaktionsgemisch (R_{G}) verstanden, dass das Reaktionsgemisch (R_{G}) neben Methylformiat, Alkaliformiat, Alkalimethanolat sowie gegebenenfalls nicht umgesetztem Methanol und nicht umgesetztem Kohlenmonoxid maximal 1 Gew.-%, bevorzugt maximal 0,5 Gew.-% weiterer Komponenten enthält, jeweils bezogen auf das Gesamtgewicht des Reaktionsgemischs (R_{G}).

Im Allgemeinen ist das molare Verhältnis von Alkaliformiat zu Alkalialkoholat im Reaktionsgemisch (R_{G}), das dem Carbonylierungsreaktor entnommen wird, größer als 1. Bevorzugt liegt das molare Verhältnis von Alkaliformiat zu Alkalialkoholat im Reaktionsgemisch (R_{G}) im Bereich von 2 bis 20, besonders bevorzugt im Bereich von 2 bis 15, ganz besonders bevorzugt im Bereich von 4 bis 12 und insbesondere im Bereich von 5 bis 10, wobei als Alkaliformiat Kaliumformiat und als Alkalialkoholat Kaliummethanolat besonders bevorzugt sind.

Die Konzentration des Alkalialkoholats, bevorzugt des Kaliummethanolats, im Reaktionsgemisch (R_{G}) beträgt im Allgemeinen mindestens 0,5 Gew.-% bezogen auf das Gesamtgewicht des Reaktionsgemischs (R_{G}). Bevorzugt liegt die Konzentration des Alkalialkoholats im Reaktionsgemisch (R_{G}) im Bereich von 0,5 bis 1,5 Gew.-%, mehr bevorzugt im Bereich von 0,5 bis 1,0 und besonders bevorzugt im Bereich von 0,55 bis 0,9 Gew.-%, jeweils bezogen auf das Gesamtgewicht des Reaktionsgemischs (R_{G}).

Die Konzentration des Alkalialkoholats, bevorzugt des Kaliummethanolats, im Reaktionsgemisch (R_{G}) beträgt im Allgemeinen mindestens 0,5 Gew.-% bezogen auf das Gesamtgewicht des Reaktionsgemischs (R_{G}). Bevorzugt beträgt die Konzentration des Alkalialkoholats im Reaktionsgemisch (R_{G}) > 0,5 Gew.-%, besonders bevorzugt mindestens 0,51 Gew.-%, bezogen auf das Gesamtgewicht des Reaktionsgemischs (R_{G}). Bevorzugt liegt die Konzentration des Alkalialkoholats im Reaktionsgemisch (R_{G}) im Bereich von > 0,5 bis 1,5 Gew.-%, mehr bevorzugt im Bereich von > 0,5 bis 1,0 und besonders bevorzugt im Bereich von 0,51 bis 0,9 Gew.-%, insbesondere im Bereich von 0,55 bis 0,9 Gew.-%, jeweils bezogen auf das Gesamtgewicht des Reaktionsgemischs (R_{G}).

Die Konzentration des Alkaliformiats, bevorzugt des Kaliumformiats, im Reaktionsgemisch (R_{G}) beträgt im Allgemeinen mindestens 2,25 Gew.-% bezogen auf das Gesamtgewicht des Reaktionsgemischs (R_{G}). Bevorzugt liegt die Konzentration des Alkaliformiats im Bereich von 2,5 bis 15 Gew.-%, bevorzugt im Bereich von 3 bis 10 Gew.-% und besonders bevorzugt im Bereich von 5 bis 7,5 Gew.-%, jeweils bezogen auf das Gesamtgewicht des Reaktionsgemischs (R_{G}).

Die vorstehenden Gew.-%-Angaben für die Konzentrationen des Alkaliformiats und des Alkalimethanolats im Reaktionsgemisch (R_{G}) gelten unter der Voraussetzung, dass das molare Verhältnis von Alkaliformiat zu Alkalialkoholat im Reaktionsgemisch (R_{G}) größer als 1 ist.

In einer besonders bevorzugten Ausführungsform wird beim erfindungsgemäßen Verfahren ein Reaktionsgemisch (R_{G}) erhalten, wobei das Reaktionsgemisch (R_{G}) 0,5 bis 1,5 Gew.-% Alkalialkoholat und 2,5 bis 15 Gew.-% Alkaliformiat, jeweils bezogen auf das Gesamtgewicht des Reaktionsgemischs (R_{G}), enthält.

In einer besonders bevorzugten Ausführungsform wird beim erfindungsgemäßen Verfahren ein Reaktionsgemisch (R_{G}) erhalten, wobei das Reaktionsgemisch (R_{G}) 0,51 bis 1,5 Gew.-% Alkalialkoholat und 2,5 bis 15 Gew.-% Alkaliformiat, jeweils bezogen auf das Gesamtgewicht des Reaktionsgemischs (R_{G}), enthält.

Das Reaktionsgemisch (R_{G}) enthält im Allgemeinen maximal 48 Gew.-% Methylformiat, bezogen auf das Gesamtgewicht des Reaktionsgemischs (R_{G}). Bevorzugt enthält das Reaktionsgemisch (R_{G}) 12 bis 45 Gew.-% Methylformiat, mehr bevorzugt 25 bis 45 Gew.-% und insbesondere 35 bis 45 Gew.-%, jeweils bezogen auf das Gesamtgewicht des Reaktionsgemischs (R_{G}).

Das Reaktionsgemisch (R_{G}) enthält im Allgemeinen nicht umgesetztes Methanol. Bevorzugt enthält das Reaktionsgemisch (R_{G}) 40 bis 85 Gew.-% Methanol, mehr bevorzugt 45 bis 60 Gew.-% und insbesondere 45 bis 55 Gew.-%, jeweils bezogen auf das Gesamtgewicht des Reaktionsgemischs (R_{G}).

In einer bevorzugten Ausführungsform des erfindungsgemäßen Verfahrens enthält das Reaktionsgemisch (R_{G})

| | |
|---|---|
| 12 bis 45 Gew.-% | Methylformiat, |
| 40 bis 85 Gew.-% | Methanol, |
| 2,5 bis 15 Gew.-% | Alkaliformiat, |
| 0,5 bis 1,5 Gew.-% | Alkalialkoholat und |
| 0 bis 2 Gew.-% | Kohlenmonoxid, |

wobei die Summe aller im Reaktionsgemisch (R_{G}) enthaltenen Komponenten 100 Gew.-% ergibt und das Reaktionsgemisch (R_{G}) maximal 1 Gew.-%, bevorzugt maximal 0,5 Gew.-% weiterer Komponenten enthält, die von Methylformiat, Methanol, Alkaliformiat, Alkalialkoholat, Kohlenmonoxid und Wasser verschieden sind, jeweils bezogen auf das Gesamtgewicht des Reaktionsgemischs (R_{G}).

In einer besonders bevorzugten Ausführungsform des erfindungsgemäßen Verfahrens enthält das Reaktionsgemisch (R_{G})

| | |
|---|---|
| 35 bis 45 Gew.-% | Methylformiat, |
| 45 bis 62 Gew.-% | Methanol, |
| 2,5 bis 7,5 Gew.-% | Kaliumformiat, |
| 0,5 bis 0,8 Gew.-% | Kaliummethanolat und |
| 0 bis 1,5 Gew.-% | Kohlenmonoxid, |

wobei die Summe aller im Reaktionsgemisch (R_{G}) enthaltenen Komponenten 100 Gew.-% ergibt und das Reaktionsgemisch (R_{G}) maximal 1 Gew.-%, bevorzugt maximal 0,5 Gew.-% weiterer Komponenten enthält, die von Methylformiat, Methanol, Kaliumformiat, Kaliummethanolat, Kohlenmonoxid und Wasser verschieden sind, jeweils bezogen auf das Gesamtgewicht des Reaktionsgemischs (R_{G}).

Weitere Komponenten, die in den vorstehenden Mengen im Reaktionsgemisch (R_{G}) enthalten sein können, sind beispielsweise Verunreinigungen in den Einsatzstoffen, wie Stickstoff, Argon, Wasserstoff oder Methan aus dem eingesetzten Kohlenmonoxid und Verunreinigungen, wie Formaldehyd und Formaldehyddimethylacetal aus dem eingesetzten Methanol sowie Verunreinigungen, die in den eingesetzten Alkoholaten und Formiaten enthalten sind, und Nebenprodukte der Methanolcarbonylierung, wie Dimethylether und Methylglyoxalmethylhemiacetal.

In einer bevorzugten Ausführungsform setzt sich der dem Carbonylierungsreaktor zugeführte flüssige Feed im Wesentlichen aus Methanol, Alkalialkoholat und Alkaliformiat sowie gegebenenfalls Methylformiat zusammen. In einer bevorzugten Ausführungsform enthält der flüssige Feed maximal 1 Gew.-%, bevorzugt maximal 0,5 Gew.-% von Methanol, Alkalimethanolat und Alkaliformiat sowie gegebenenfalls Methylformiat verschiedenen Komponenten, jeweils bezogen auf das Gesamtgewicht des flüssigen Feeds.

Unter flüssigem Feed sind vorliegend alle dem Reaktor zugeführten flüssigen Komponenten zu verstehen, also die Summe aus frisch zugeführten und rückgeführten flüssigen Komponenten.

Die Zusammensetzung des Reaktionsgemischs (R_{G}) lässt sich über die vorstehend aufgeführten Feedverhältnisse von Kohlenmonoxid, Methanol und Katalysatorsystem (Alkaliformiat und Alkalialkoholat) steuern.

Die Menge des im Reaktionsgemisch (R_{G}) enthaltenen Methylformiats setzt sich zusammen aus der Menge Methylformiat, die im Carbonylierungsreaktor aus Kohlenmonoxid und Methanol gebildet wird, sowie aus der Menge Methylformiat, die gegebenenfalls aus einem nachgelagerten Aufarbeitungsschritt zum Carbonylierungsreaktor rückgeführt wird.

Die Menge des im Reaktionsgemisch (R_{G}) enthaltenen Kohlenmonoxids und Methanols wird über die Feedverhältnisse von Kohlenmonoxid zu Methanol sowie über den Umsatz von Kohlenmonoxid mit Methanol zu Methylformiat im Carbonylierungsreaktor sowie über den Reaktionsdruck der Carbonylierung gesteuert.

Die Einstellung der Zusammensetzung des Reaktionsgemischs (R_{G}) kann dabei durch übliche, dem Fachmann bekannte Regelungsmethoden erfolgen, beispielsweise durch eine Messeinheit, die die Zusammensetzung des Reaktionsgemisch (R_{G}), das aus dem Carbonylierungsreaktor entnommen wird misst, und bei einer Abweichung der Sollzusammensetzung die Feedverhältnisse entsprechend anpasst.

Die Mengen des im Reaktionsgemisch (R_{G}) enthaltene Alkaliformiats und Alkalialkoholats werden über die dem Carbonylierungsreaktor zugeführten Mengen an Alkaliformiat und Alkalialkoholat gesteuert, das heißt über Menge und Zusammensetzung des Katalysatorsystems. Alkaliformiat und Alkalialkoholat werden dem Carbonylierungsreaktor im Allgemeinen als Lösung zugeführt, bevorzugt gelöst in Methanol.

Das Katalysatorsystem, enthaltend Alkaliformiat und Alkalialkoholat, kann dem Carbonylierungsreaktor von außen frisch zugeführt werden. In einer bevorzugten Ausführungsform wird das erfindungsgemäße Verfahren kontinuierlich durchgeführt und das im Katalysatorsystem enthaltende Alkaliformiat und Alkalialkoholat wird zum Carbonylierungsreaktor aus einem nachgelagerten Aufarbeitungsschritt rückgeführt.

Hierbei ist zu beachten, dass das im Katalysatorsystem enthaltene Alkalialkoholat im Carbonylierungsreaktor teilweise zu Alkaliformiat umgesetzt wird. Dies erfolgt durch Reaktion von Alkalialkoholat mit Methylformiat zu Alkaliformiat und Dimethylether und durch Hydrolyse von Alkalialkoholat und Methylformiat mit Wasserspuren zu Alkaliformiat und Methanol. Die Bildung von Alkaliformiat wird anhand der nachfolgenden Reaktionsgleichungen iii) bis v) am Beispiel der Bildung von Kaliumformiat aus Kaliummethanolat beschrieben

iii) HCOOCH₃ + KOCH₃ → HCOOK + CH₃OCH₃

iv) KOCH₃ + H₂O → KOH + CH₃OH

v) KOH + HCOOCH₃ → HCOOK + CH₃OH

Um die erfindungsgemäße Zusammensetzung von Alkaliformiat und Alkalialkoholat im Reaktionsgemisch (R_{G}) einzustellen, ist es daher im Allgemeinen erforderlich, aus dem Reaktionsgemisch (R_{G}) einen Teil des gemäß der Reaktionsgleichungen iii) bis v) gebildeten Alkaliformiats abzutrennen. Darüber hinaus ist es im Allgemeinen erforderlich, das gemäß den Reaktionsgleichungen iii) und iv) abreagierte Alkalimethanolat zu ersetzen.

Die Abtrennung des gebildete Alkaliformiats und das Ersetzten des abreagierten Alkalialkoholats kann dabei sequenziell oder kontinuierlich erfolgen. Bevorzugt wird das gebildete Alkaliformiat kontinuierlich abgetrennt und das abreagierte Alkalialkoholat kontinuierlich durch frisch von außen zugeführtes Alkalialkoholat ersetzt.

In einer besonders bevorzugten Ausführungsform wird das aus dem Carbonylierungsreaktor entnommene Reaktionsgemisch (R_{G}) weiter aufgearbeitet, umfassend die folgenden Schritte
(a) Abtrennung von Kohlenmonoxid aus dem Reaktionsgemisch (RG) in einer Trennvorrichtung unter Erhalt eines Gasstroms (G1), der Kohlenmonoxid enthält, und eines flüssigen Stroms (L1), der Methylformiat, Alkaliformiat, Alkalialkoholat und Methanol enthält,
(b) Abtrennung des Methylformiats aus dem flüssigen Strom (L1) in einer ersten Destillationsvorrichtung unter Erhalt eines Destillats (D1), das Methylformiat enthält, und eines Sumpfgemischs (S1), das Alkaliformiat, Alkalialkoholat und Methanol enthält,
(c) Aufteilen des Sumpfgemischs (S1) in einen Teilstrom (S1a), der zum Carbonylierungsreaktor rückgeführt wird, und einen Teilstrom (S1b)und
(d) Abtrennung des Methanols aus dem Teilstrom (S1b) in einer zweiten Destillationsvorrichtung unter Erhalt eines Destillats (D2), das Methanol enthält und zur ersten Destillationsvorrichtung rückgeführt wird, und eines Sumpfgemischs (S2), das Alkaliformiat und Alkalialkoholat enthält.

Die Abtrennung des Kohlenmonoxids in Schritt (a) ist nicht zwingend erforderlich. Es ist auch möglich, das Reaktionsgemisch (R_{G}) direkt der ersten Destillationsvorrichtung in Schritt (b) zuzuführen. Für den Fall, das Kohlenmonoxid aus dem Reaktionsgemisch (R_{G}) abgetrennt wird, wird der Gasstrom (G1) bevorzugt zum Carbonylierungsreaktor rückgeführt. Bei der Abtrennung des Kohlenmonoxids in Schritt a) wird als Trennvorrichtung bevorzugt eine Flashvorrichtung eingesetzt.

In Schritt (b) kann aus dem flüssigen Strom (L1) bzw. aus dem Reaktionsgemisch (R_{G}) das Methylformiat vollständig oder teilweise abgetrennt werden.

Bei der teilweisen Abtrennung werden beispielsweise 50 bis 90 Gew.-% des im flüssigen Strom (L1) beziehungsweise des im Reaktionsgemisch (R_{G}) enthaltenen Methylformiats, bevorzugt 60 bis 90 Gew.-% und mehr bevorzugt 80 bis 90 Gew.-% abgetrennt, jeweils bezogen auf das Gesamtgewicht des im flüssigen Strom (L1) beziehungsweise des im Reaktionsgemisch (R_{G}) enthaltenen Methylformiats.

In einer weiteren Ausführungsform des erfindungsgemäßen Verfahrens wird in der ersten Destillationsvorrichtung in Verfahrensstufe (b) das Methylformiat vollständig abgetrennt. Unter "vollständig abgetrennt" wird im Rahmen der vorliegenden Erfindung eine Abtrennung von mehr als 90 % des im flüssigen Strom (L1) bzw. im Reaktionsgemisch (R_{G}) enthaltenen Methylformiats, bevorzugt mehr als 98,5 %, besonders bevorzugt mehr als 99 %, insbesondere mehr als 99,5 %, verstanden, jeweils bezogen auf die Gesamtmenge des im flüssigen Strom (L1) beziehungsweise des im Reaktionsgemisch (R_{G}) enthaltenen Methylformiats.

Bei der teilweisen Abtrennung des Methylformiats enthält das Sumpfgemisch (S1) noch Methylformiat. Bei der vollständigen Abtrennung des Methylformiats ist das Sumpfgemisch (S1) weitestgehend frei von Methylformiat. Die vollständige Abtrennung des Methylformiats ist bevorzugt.

Die Abtrennung des Methylformiats kann beispielsweise in einem Verdampfer oder in einer Destillationseinheit bestehend aus Verdampfer und Kolonne, wobei die Kolonne mit Packungen, Füllkörper und/oder Böden gefüllt ist, erfolgen.

Das gemäß Schritt (b) abgetrennte Destillat (D1) kann neben Methylformiat noch Methanol enthalten. In einer bevorzugten Ausführungsform enthält das Destillat (D1) 60 bis 97 Gew.-% Methylformiat und 3 bis 40 Gew.-% Methanol, bevorzugt 75 bis 85 Gew.-% Methylformiat und 15 bis 25 Gew.-% Methanol. Das Destillat (D1) kann weiter aufgearbeitet, beispielsweise durch Destillation. Das dabei abgetrennte Methanol kann zum Carbonylierungsreaktor rückgeführt werden. Das rückgeführte Methanol kann Methylformiat enthalten.

Es ist auch möglich, das Methylformiat weiter umzusetzen. Das Methylformiat kann beispielsweise zu Ameisensäure hydrolysiert werden. Das dabei entstehende Methanol kann ebenfalls zum Carbonylierungsreaktor rückgeführt werden. Das rückgeführte Methanol kann Methylformiat enthalten.

Bei der Abtrennung des Methylformiats in Schritt (b) wird ein Sumpfgemisch (S1) erhalten, das Alkaliformiat, Alkalialkoholat und Methanol enthält. Im Sumpfgemisch (S1) liegen Alkaliformiat und Alkalialkoholat bevorzugt gelöst in Methanol vor.

Die Steuerung der im Reaktionsgemisch (R_{G}) enthaltenen Mengen an Alkaliformiat und Alkalialkoholat erfolgt in einer bevorzugten Ausführungsform durch die Aufteilung des Sumpfgemisches (S1) in die Teilströme (S1a) und (S1b).

Die Abtrennung des gebildeten Alkaliformiats aus dem Reaktionsgemisch (RG) erfolgt über den Teilstrom (S1b). Über die Menge des abgetrennten Teilstroms (S1b) wird die Aufpegelung des Alkaliformiats im Reaktionsgemisch (R_{G}) gesteuert. Zur Ausschleusung des gebildeten Alkaliformiats wird der Teilstrom (S1b) der zweiten Destillationsvorrichtung zugeleitet. Um die erfindungsgemäße Zusammensetzung des Reaktionsgemischs (R_{G}) einzustellen wird das Sumpfgemisch (S1) derart aufgeteilt, dass das Gewichtsmengenverhältnis des Teilstroms (S1a) zu dem Teilstrom (S1b) größer als 50 zu 1 ist, bevorzugt größer als 100 zu 1.

Anders ausgedrückt bedeutet dies, dass pro ausgeschleusten Gewichtsmengenteil Teilstrom (S1b) mindestens 50 Gewichtmengenanteile Teilstrom (S1a), bevorzugt mindestens 100 Gewichtsmengenanteile Teilstrom (S1 a), zum Carbonylierungsreaktor rückgeführt werden.

Im Sumpf der zweiten Destillationsvorrichtung wird ein Sumpfgemisch (S2) erhalten, das Alkaliformiat und Alkalimethanolat enthält. Um die Ausfällung von Feststoffen zu verhindern und um die Gefahr von Verkrustungen oder Verstopfungen im Sumpf der zweiten Destillationsvorrichtung zu minimieren, wird der zweiten Destillationsvorrichtung in einer bevorzugten Ausführungsform Wasser zugegeben. Das Wasser kann beispielsweise als Dampf oder Dampfkondensat der zweiten Destillationsvorrichtung zugeführt werden.

In dieser Ausführungsform umfasst die Aufarbeitung des Reaktionsgemischs (R_{G}) die folgenden Schritte:
(a) Abtrennung von Kohlenmonoxid aus dem Reaktionsgemisch (R_{G}) in einer Trennvorrichtung unter Erhalt eines Gasstroms (G1), der Kohlenmonoxid enthält, und eines flüssigen Stroms (L1), der Methylformiat, Alkaliformiat, Alkalialkoholat und Methanol enthält,
(b) Abtrennung des Methylformiats aus dem flüssigen Strom (L1) in einer ersten Destillationsvorrichtung unter Erhalt eines Destillats (D1), das Methylformiat enthält, und eines Sumpfgemischs (S1), das Alkaliformiat, Alkalialkoholat und Methanol enthält,
(c) Aufteilen des Sumpfgemischs (S1) in einen Teilstrom (S1a), der zum Carbonylierungsreaktor rückgeführt wird, und einen Teilstrom (S1b)und
(d) Abtrennung des Methanols aus dem Teilstrom (S1b) in einer zweiten Destillationsvorrichtung, der Wasser zugegeben wird, unter Erhalt eines Destillats (D2), das Methanol enthält und zur ersten Destillationsvorrichtung rückgeführt wird, und eines Sumpfgemischs (S2w) das Alkalihydroxid, Alkaliformiat und Wasser enthält.

Das Wasser kann beispielsweise als Dampf oder Dampfkondensat zur zweiten Destillationsvorrichtung zugeführt werden. Die Zugabe des Wassers erfolgt dabei in einer bevorzugten Ausführungsform im Sumpf der zweiten Destillationsvorrichtung.

Die Zugabe von Wasser führt dazu, dass das Alkalialkoholat zu dem entsprechenden Alkalihydroxid und dem entsprechenden Alkohol hydrolysiert wird. Für den Fall des bevorzugten Kaliummethanolats wird dieses zu Kaliumhydroxid und Methanol hydrolysiert. Das so gebildete Methanol wird ebenfalls als Destillat (D2) am Kopf der zweiten Destillationsvorrichtung abgetrennt und bevorzugt zur ersten Destillationsvorrichtung rückgeführt.

Da über dem Teilstrom (S1b) neben dem Alkaliformiat auch Alkalialkoholat abgetrennt wird, muss dem Carbonylierungsreaktor frisches Alkalialkoholat von außen zugeführt werden. Die Zuführung des Alkalialkoholats von außen zum Carbonylierungsreaktor erfolgt in einer bevorzugten Ausführungsform kontinuierlich. Das frisch zugeführte Alkalialkoholat kann dabei als Feststoff, bevorzugt jedoch gelöst in Methanol dem Carbonylierungsreaktor zugeführt werden. Das frisch zugeführte Alkalialkoholat kann dem Carbonylierungsreaktor als getrennter Strom zugeführt werden. Es ist auch möglich das frisch zugeführte Alkalialkoholat dem aus Schritt (c) zum Carbonylierungsreaktor rückgeführten Teilstrom (S1a) zuzumischen.

Die vorliegende Erfindung wird durch die nachfolgenden Figuren und die nachfolgenden Beispiele verdeutlicht, ohne sie hierauf zu beschränken.

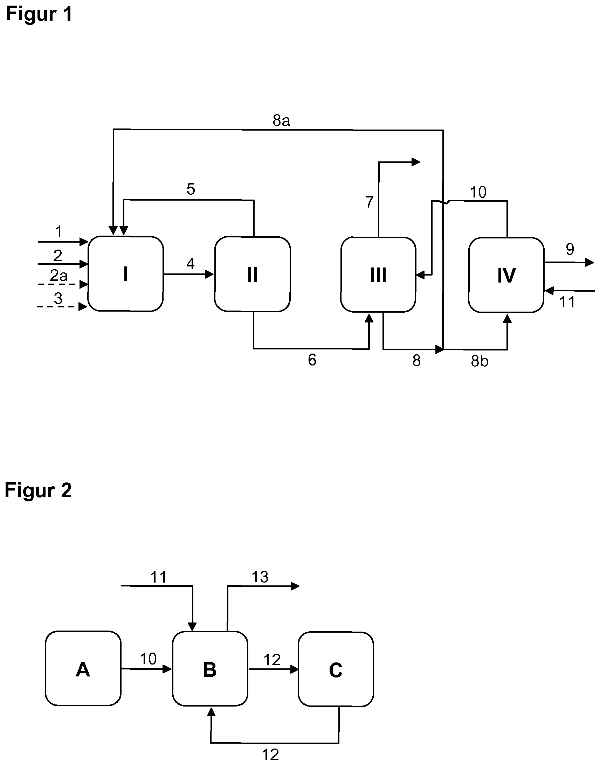

Figur 1 zeigt ein Blockdiagramm einer bevorzugten Ausführungsform des erfindungsgemäßen Verfahrens. In Figur 1 haben die Bezugszeichen die folgenden Bedeutungen:
- 1: Strom enthaltend Kohlenmonoxid
- 2: Strom enthaltend Methanol
- 2a: Strom enthaltend Methanol, der aus einer nachgelagerten Aufarbeitungsstufe rückgeführt wird
- 3: Strom enthaltend Alkalialkoholat gelöst in Methanol
- 4: Strom enthaltend Methylformiat, Alkaliformiat, Alkalialkoholat sowie gegebenenfalls nicht umgesetztes Methanol und nicht umgesetztes Kohlenmonoxid; entspricht Reaktionsgemisch (R_{G})
- 5: Strom enthaltend Kohlenmonoxid; entspricht Gasstrom (G1)
- 6: Strom enthaltend Methylformiat, Alkaliformiat, Alkalialkoholat und Methanol; entspricht flüssigem Strom (L1)
- 7: Strom enthaltend Methylformiat sowie gegebenenfalls Methanol; entspricht Destillat (D1)
- 8: Strom enthaltend Alkaliformiat, Alkalialkoholat und Methanol; entspricht Sumpfgemisch (S1)
- 8a: Strom enthaltend Alkaliformiat, Alkalialkoholat und Methanol; entspricht Teilstrom (S1a)
- 8b: Strom enthaltend Alkaliformiat, Alkalialkoholat und Methanol; entspricht Teilstrom (S1b)
- 9: Strom enthaltend Alkaliformiat, Alkalihydroxid und Wasser; entspricht Sumpfgemisch (S2w)
- 10: Strom enthaltend Methanol; entspricht Destillat (D2)
- 11: Strom enthaltend Wasser

- I: Carbonylierungsreaktor
- II: Trennvorrichtung
- III: erste Destillationsvorrichtung
- IV: zweite Destillationsvorrichtung

Dem Carbonylierungsreaktor I werden Kohlenmonoxid, Methanol und das Katalysatorsystem, enthaltend Alkaliformiat und Alkalialkoholat, zugeführt. Im Carbonylierungsreaktor wird dabei Methanol mit Kohlenmonoxid zu Methylformiat umgesetzt, wobei das Reaktionsgemisch (R_{G}) erhalten wird. Die Zuführung des Kohlenmonoxids erfolgt dabei über den Strom 1 (von außen frisch zugeführtes Kohlenmonoxid) und den Strom 5 (von der Trennvorrichtung II rückgeführtes Kohlenmonoxid). Das Alkaliformiat und das Alkalialkoholat werden als Strom 8a (entspricht Teilstrom S1a) aus dem Sumpf der ersten Destillationsvorrichtung III zum Carbonylierungsreaktor rückgeführt. Der Strom 8a enthält dabei Methanol, das Alkaliformiat und das Alkalialkoholat, bevorzugt in gelöster Form.

Verluste an Alkalialkoholat können gegebenenfalls über den Strom 3 ausgeglichen werden. Strom 3 enthält bevorzugt Alkalialkoholat gelöst in Methanol. Methanol wird dem Carbonylierungsreaktor bevorzugt über die Ströme 2 beziehungsweise 2a zugeführt. Strom 2 beschreibt den Fall der Zuführung von frischem Methanol von außen. Es ist jedoch auch möglich alternativ oder zusätzlich das Methanol über den Strom 2a zuzuführen, der aus späteren Aufarbeitungsstufen oder der Umsetzungen des Methylformiats entstammt.

Im Carbonylierungsreaktor I wird ein Reaktionsgemisch (R_{G}) erhalten, das aus dem Carbonylierungsreaktor I entnommen wird und als Strom 4 zu der Trennvorrichtung II weitergeleitet wird. In der Trennvorrichtung II wird aus dem Reaktionsgemisch (R_{G}) nicht umgesetztes Kohlenmonoxid abgetrennt. Dies erfolgt bevorzugt durch Entspannung des Reaktionsgemischs (R_{G}). In der Trennvorrichtung II wird ein Gasstrom (G1) erhalten, der im Wesentlichen aus Kohlenmonoxid besteht und als Strom 5 zum Carbonylierungsreaktor I rückgeführt wird.

Aus der Trennvorrichtung II wird ein flüssiger Strom (L1) entnommen, der Methylformiat, Alkaliformiat, Alkalialkoholat und Methanol enthält und als Strom 6 zur ersten Destillationsvorrichtung III weitergeleitet wird. In der ersten Destillationsvorrichtung III wird am Kopf dieser Destillationsvorrichtung ein Strom 7 abgetrennt, der Methylformiat enthält (Destillat (D1)). Am Kopf der ersten Destillationsvorrichtung III kann auch eine Mischung aus Methylformiat und Methanol abgetrennt werden. In einer bevorzugten Ausführungsform wird der Strom 7 weiter umgesetzt. Bevorzugt wird das Methylformiat zu Ameisensäure hydrolysiert. Das bei der Hydrolyse entstehende Methanol kann als Strom 2a zum Carbonylierungsreaktor I rückgeführt werden. Im Sumpf der ersten Destillationsvorrichtung III wird ein Sumpfgemisch (S1) erhalten, das Alkaliformiat, Alkalialkoholat und Methanol enthält. Das Sumpfgemisch (S1) wird als Strom 8 aus der Destillationsvorrichtung III entnommen. Der Strom 8 wird aufgeteilt in einen Teilstrom 8a und einen Teilstrom 8b. Der Teilstrom 8a (entspricht Teilstrom S1a) wird zum Carbonylierungsreaktor I rückgeführt. Der Strom 8b (entspricht Teilstrom S1b) wird in der zweiten Destillationsvorrichtung IV weiter aufgearbeitet. Der zweiten Destillationsvorrichtung IV wird als Strom 11 Wasser zugeführt. Die Zufuhr von Strom 11 erfolgt bevorzugt in den Sumpf der zweiten Destillationsvorrichtung IV. In der zweiten Destillationsvorrichtung IV wird das im Strom 8b enthaltene Alkalialkoholat zum entsprechenden Alkalihydroxid und dem entsprechenden Alkohol hydrolysiert. Bei dem bevorzugt in Strom 8b enthaltenen Kaliummethanolat entsteht bei der Hydrolyse Kaliumhydroxid und Methanol. Am Kopf der zweiten Destillationsvorrichtung IV wird Methanol als Strom 10 entnommen und zur ersten Destillationsvorrichtung III rückgeführt. Als Strom 9 wird aus der zweiten Destillationsvorrichtung IV eine wässrige Lösung entnommen, die Kaliumhydroxid, Kaliumformiat und Wasser enthält.

Figur 2 zeigt eine Laborapparatur, in der das erfindungsgemäße Verfahren durchgeführt wird. In Figur 2 haben die Bezugszeichen die folgenden Bedeutungen:
- A: Vorlagebehälter enthaltend ein Gemisch aus Methanol, Kaliummethanolat und gegebenenfalls einem Alkaliformiat
- B: Carbonylierungsreaktor
- C: Online ATR-FIR-Messsonde (ATR-MIR, Matrix MF von der Firma Bruker)
- 10: Strom enthaltend Methanol, Kaliummethanolat und gegebenenfalls ein Alkaliformiat
- 11: Strom enthaltend Kohlenmonoxid
- 12: Strom enthaltend Methylformiat, gegebenenfalls Alkaliformiat, Kaliummethanolat, Methanol und gegebenenfalls nicht umgesetztes Kohlenmonoxid
- 13: Strom enthaltend Methylformiat, gegebenenfalls Alkaliformiat, Kaliummethanolat, Methanol und gegebenenfalls nicht umgesetztes Kohlenmonoxid; entspricht Reaktionsgemisch (R_{G}).

Die Erfindung wird im Folgenden anhand von Ausführungsbeispielen erläutert, ohne sie hierauf zu beschränken.

Die Versuche zur Carbonylierung von Methanol mit Kohlenmonoxid wurden in der in Figur 2 gezeigten Laborapparatur durchgeführt. Im Behälter A wurde ein Gemisch aus Methanol, Kaliummethanolat und gegebenenfalls einem Alkaliformiat vorgelegt. Der Carbonylierungsreaktor wurde durch einen Autoklaven aus HC-Stahl simuliert, der ein Volumen von 270 ml aufweist. Das Reaktorvolumen des Autoklaven wurde mittels eines Steigrohres in eine 150 ml Flüssigphase und eine 120 ml Gasphase getrennt. Die Beheizung erfolgte mittels Ölbad. Die Temperatur wurde durch ein Thermoelement geregelt.

Dem Carbonylierungsreaktor (B) wird aus dem Vorlagebehälter A kontinuierlich Methanol und das Katalysatorsystem zugeführt (siehe Strom 10 in Figur 2). Die Zuführung von Kohlenmonoxid erfolgt über Strom 11. Zur Analytik wird aus dem Carbonylierungsreaktor (B) ein Strom 12 entnommen, der über eine online-ATR-FIR-Messsonde zum Carbonylierungsreaktor (B) rückgeführt wird. Die ATR-FIR-Messsonde (C) weist eine Kalibrierung von 0 bis 80 Gew.-% Methylformiat in Methanol auf. In der Messsonde wurde alle 60 Sekunden ein Messpunkt ermittelt, wobei hierfür der Mittelwert aus 64 Einzelmessungen gebildet wurde.

Die Zusammensetzung des im Vorlagebehälter A enthaltenen Gemische sowie die Zusammensetzung des in den Versuchen erhalten Reaktionsgemischs (R_{G}) ist in den nachfolgenden Beispielen angegeben.

### Vergleichsbeispiel 1

Das Vergleichsbeispiel wurde in der Laboranlage gemäß Figur 2 durchgeführt. Zunächst wurde die gesamte Laboranlage mit Stickstoff inertisiert. Ebenfalls unter Stickstoff wurden 1400 g einer Lösung aus 99.2 Gew.-% Methanol und 0.8 Gew.-% Kaliummethylat (Kaliummethanolat) angesetzt und unter Stickstoff in den Vorlagebehälter (A), welcher auf einer Waage stand, überführt. 555 g/h dieser Mischung wurden als Strom (10) kontinuierlich von unten in Autoklav (B) gepumpt. 188 NI/h Kohlenmonoxid (Reinheit 99,97 Vol.-%) wurden als Strom (11) auf der Deckelseite in Carbonylisierungsreaktor zugeleitet. Autoklav (B) wurde intensiv mit 750 Umdrehungen pro Minute gerührt und bei 85°C betrieben. Das zweiphasige Reaktionsgemisch (R_{G}) wird als Strom (13) über das Steigrohr aus dem Carbonylisierungsreaktor kontinuierlich ausgetragen. Mittels eines Druckhalteventils in der Austragsleitung wurde der Druck im Carbonylisierungsreaktor bei 55 bar gehalten. Zur Online-Analytik wurden 80 I/h des Reaktionsgemischs (R_{G}) als Strom (12) mittels einer Pumpe kontinuierlich aus dem Carbonylisierungsreaktor gefördert. Strom (12) wurde auf 30°C gekühlt, anschließend über eine ATR-FIR-Sonde (C) gepumpt und von dort zurück in den Carbonylisierungsreaktor geleitet. Nach 1 Stunde wurde ein stationärer Methylformiat-Gehalt im Reaktor von 10,5 Gew.-% gemessen.

### Beispiel 2

Beispiel 2 wurde analog zum Vergleichsbeispiel 1 durchgeführt. Als Feed wurden 1400 g einer Lösung mit 96,7 Gew.-% MeOH, 0,8 Gew.-% Kaliummethylat und 2,5 Gew.-% Kaliumformiat unter Stickstoff in Vorlagebehälter (A) gefüllt. Die Versuchsdurchführung und die Versuchsparameter wurden analog zu Vergleichsbeispiel 1 durchgeführt. Nach 1 Stunde der Versuchsdurchführung wurde ein stationärer Methylformiat-Gehalt im Reaktor von 12,8 Gew.-% gemessen.

Aus Beispiel 2 ist ersichtlich, dass die Methylformiat-Bildung unter sonst gleichen Reaktionsbedingungen durch Einsatz von 2.5 Gew.-% Kaliumformiat im Feed um 22 % gesteigert werden kann.

### Beispiel 3

Beispiel 3 wurde analog zum Vergleichsbeispiel 1 durchgeführt. Als Feed wurden 1400 g einer Lösung mit 94,2 Gew.-% MeOH, 0,8 Gew.-% Kaliummethylat und 5,0 Gew.-% Kaliumformiat unter Stickstoff in Vorlagebehälter (A) gefüllt. Die Versuchsdurchführung und die Versuchsparameter wurden analog zu Vergleichsbeispiel 1 gewählt. Nach 1 Stunde der Versuchsdurchführung wurde ein stationärer Methylformiat-Gehalt im Reaktor von 13,3 Gew.-% gemessen.

Aus Beispiel 3 ist ersichtlich, dass die Methylformiat-Bildung unter sonst gleichen Reaktionsbedingungen durch Einsatz von 5 Gew.-% Kaliumformiat nochmals im Vergleich zu Beispiel 2 gesteigert werden kann.

### Vergleichsbeispiel 4

Vergleichsbeispiel 4 wurde in der Laboranlage gemäß Figur 2 durchgeführt. Zunächst wurde die gesamte Laboranlage mittels Stickstoff als Schutzgas inertisiert. Ebenfalls unter Stickstoff wurden 850 g einer Lösung aus 99.3 Gew.-% Methanol und 0.7 Gew.-% Kaliummethylat angesetzt und unter Stickstoff in Vorlagebehälter (A), welcher auf einer Waage stand, überführt. 480 g/h dieser Mischung wurden als Strom (10) kontinuierlich von unten in Autoklav (B) gepumpt. 155 NI/h Kohlenmonoxid (Reinheit 99,97 Vol.-%) wurden als Strom (11) auf der Deckelseite in den Carbonylisierungsreaktor zugeleitet. Autoklav (B) wurde intensiv mit 750 Umdrehungen pro Minute gerührt und bei 85°C betrieben. Das zweiphasige Reaktionsgemisch (R_{G}) wird als Strom (13) über das Steigrohr kontinuierlich aus dem Carbonylisierungsreaktor ausgetragen. Mittels eines Druckhalteventils in der Austragsleitung wurde der Druck im Reaktor bei 55 bar gehalten. Zur Online-Analytik wurden 80 I/h des Reaktionsgemischs (R_{G}) als Strom (12) mittels einer Pumpe kontinuierlich aus dem Carbonylisierungsreaktor (B) gefördert. Strom (12) wurde auf 30°C gekühlt, anschließend über eine ATR-FIR-Sonde (C) gepumpt und von dort zurück in den Carbonylisierungsreaktor (B) geleitet. Nach 1 Stunde wurde ein stationärer Methylformiat-Gehalt im Reaktor von 11.6 Gew.-% gemessen.

### Beispiel 5

Beispiel 5 wurde ebenfalls in der Laboranlage gemäß Figur 2 durchgeführt. Als Feed wurden 850 g einer Lösung mit 94,3 Gew.-% MeOH, 0,7 Gew.-% Kaliummethylat und 5,0 Gew.-% Natriumformiat unter Stickstoff in Vorlagebehälter (A) gefüllt. Die Versuchsdurchführung und die Versuchsparameter wurden analog zu Vergleichsbeispiel 4 gewählt. Nach 1 Stunde der Versuchsdurchführung wurde ein stationärer Methylformiat-Gehalt im Reaktor von 13,0 Gew.-% gemessen.

### Beispiel 6

Beispiel 6 wurde ebenfalls in der Laboranlage gemäß Figur 2 durchgeführt. Als Feed wurden 850 g einer Lösung mit 94,3 Gew.-% MeOH, 0,7 Gew.-% Kaliummethylat und 5,0 Gew.-% Kaliumformiat unter Stickstoff in Vorlagebehälter (A) gefüllt. Die Versuchsdurchführung und die Versuchsparameter wurden analog zu Vergleichsbeispiel 4 gewählt. Nach 1 Stunde der Versuchsdurchführung wurde ein stationärer Methylformiat-Gehalt im Reaktor von 15,2 Gew.-% gemessen.

### Beispiel 7

Beispiel 7 wurde ebenfalls in der Laboranlage gemäß Figur 2 durchgeführt. Als Feed wurden 850 g einer Lösung mit 94,3 Gew.-% MeOH, 0,7 Gew.-% Kaliummethylat und 5,0 Gew.-% Rubidiumformiat unter Stickstoff in Vorlagebehälter (A) gefüllt. Die Versuchsdurchführung und die Versuchsparameter wurden analog zu Vergleichsbeispiel 4 gewählt. Nach 1 Stunde der Versuchsdurchführung wurde ein stationärer Metyhlformiat-Gehalt im Reaktor von 13,6 Gew.-% gemessen.

Aus den Beispielen 5 bis 7 ist ersichtlich, dass durch Zugabe von 5 Gew.-% Natriumformiat, Kaliumformiat und Rubidiumformiat die Methylformiat-Bildung unter sonst gleichen Reaktionsbedingungen im Vergleich zu Vergleichsbeispiel 4 deutlich gesteigert werden kann. Bevorzugt ist jedoch die Verwendung von Kaliumformiat (Beispiel 6).

## Patentansprüche

1. Verfahren zur Herstellung von Methylformiat durch Carbonylierung von Methanol mit Kohlenmonoxid in einem Carbonylierungsreaktor in Gegenwart eines Katalysatorsystems, das Alkaliformiat und Alkalialkoholat enthält, unter Erhalt eines Reaktionsgemischs (R_{G}), das Methylformiat, Alkaliformiat, Alkalialkoholat sowie gegebenenfalls nicht umgesetztes Methanol und nicht umgesetztes Kohlenmonoxid enthält, und aus dem Carbonylierungsreaktor entnommen wird, wobei das Reaktionsgemisch (R_{G}) mindestens 0,5 Gew.-% Alkalialkoholat bezogen auf das Gesamtgewicht des Reaktionsgemischs (R_{G}) enthält und das molare Verhältnis von Alkaliformiat zu Alkalialkoholat im Reaktionsgemisch (R_{G}) größer als 1 ist.

2. Verfahren gemäß Anspruch 1, wobei die Alkalimetallkomponenten des Alkaliformiats und des Alkalialkoholats unabhängig voneinander ausgewählt sind aus der Gruppe bestehend aus Lithium, Natrium, Kalium, Rubidium und Cäsium.

3. Verfahren gemäß Anspruch 1 oder 2, wobei das Alkaliformiat ausgewählt ist aus der Gruppe bestehend aus Natriumformiat und Kaliumformiat und das Alkalialkoholat ausgewählt ist aus der Gruppe bestehend aus Natriumalkoholat und Kaliumalkoholat.

4. Verfahren gemäß einem der Ansprüche 1 bis 3, wobei die Alkalimetallkomponente des Alkaliformiats und des Alkalialkoholats identisch sind.

5. Verfahren gemäß einem der Ansprüche 1 bis 4, wobei das Alkaliformiat Kaliumformiat ist und das Alkalialkoholat Kaliummethanolat ist.

6. Verfahren gemäß einem der Ansprüche 1 bis 5, wobei das eingesetzte Methanol maximal 250 Gew.-ppm, bevorzugt maximal 100 Gew.-ppm und besonders bevorzugt maximal 50 Gew.-ppm Wasser enthält, jeweils bezogen auf das Gesamtgewicht des eingesetzten Methanols.

7. Verfahren gemäß einem der Ansprüche 1 bis 6, wobei das Reaktionsgemisch (R_{G}) 0,5 bis 1,5 Gew.-% Alkalialkoholat und 2,5 bis 12 Gew.-% Alkaliformiat enthält, jeweils bezogen auf das Gesamtgewicht des Reaktionsgemischs (R_{G}).

8. Verfahren gemäß einem der Ansprüche 1 bis 7, wobei das Reaktionsgemisch (R_{G}) weiter aufgearbeitet wird, umfassend die folgenden Schritte
(a) Abtrennung von Kohlenmonoxid aus dem Reaktionsgemisch (R_{G}) in einer Trennvorrichtung unter Erhalt eines Gasstroms (G1), der Kohlenmonoxid enthält, und eines flüssigen Stroms (L1), der Methylformiat, Alkaliformiat, Alkalialkoholat und Methanol enthält,
(b) Abtrennung des Methylformiats aus dem flüssigen Strom (L1) in einer ersten Destillationsvorrichtung unter Erhalt eines Destillats (D1), das Methylformiat enthält, und eines Sumpfgemischs (S1), das Alkaliformiat, Alkalialkoholat und Methanol enthält,
(c) Aufteilen des Sumpfgemischs (S1) in einen Teilstrom (S1a), der zum Carbonylierungsreaktor rückgeführt wird, und einen Teilstrom (S1 b) und
(d) Abtrennung des Methanols aus dem Teilstrom (S1b) in einer zweiten Destillationsvorrichtung unter Erhalt eines Destillats (D2), das Methanol enthält und zur ersten Destillationsvorrichtung rückgeführt wird, und eines Sumpfgemischs (S2), das Alkaliformiat und Alkalialkoholat enthält.

9. Verfahren gemäß einem der Ansprüche 1 bis 8, wobei das Gewichtsmengenverhältnis des Teilstroms (S1a) zu Teilstrom (S1b) größer als 50 zu 1 ist.

10. Verfahren gemäß einem der Ansprüche 1 bis 9, wobei das molare Verhältnis von Alkaliformiat zu Alkalialkoholat im Reaktionsgemisch (R_{G}) größer 2, besonders bevorzugt größer 3 und insbesondere bevorzugt größer 5 ist.

11. Verfahren gemäß einem der Ansprüche 1 bis 10, wobei das Reaktionsgemisch (R_{G})
| | |
|---|---|
| 12 bis 45 Gew.-% | Methylformiat, |
| 40 bis 85 Gew.-% | Methanol, |
| 2,5 bis 15 Gew.-% | Alkaliformiat, |
| 0,5 bis 1,5 Gew.-% | Alkalialkoholat und |
| 0 bis 2 Gew.-% | Kohlenmonoxid |
enthält.

12. Verwendung einer Mischung, die ein Alkaliformiat und ein Alkalialkoholat enthält, in der das molare Verhältnis von Alkaliformiat zu Alkalialkoholat größer als 1 ist, als Katalysatorsystem zur Herstellung von Methylformiat durch Carbonylierung von Methanol mit Kohlenmonoxid.

13. Verwendung einer Mischung gemäß Anspruch 12, wobei das molare Verhältnis von Alkaliformiat zu Alkalialkoholat größer als 2 ist.

## Claims

1. A process for preparing methyl formate by carbonylation of methanol by means of carbon monoxide in a carbonylation reactor in the presence of a catalyst system comprising alkali metal formate and alkali metal alkoxide to give a reaction mixture (R_{M}) which comprises methyl formate, alkali metal formate, alkali metal alkoxide and possibly unreacted methanol and unreacted carbon monoxide and is taken from the carbonylation reactor, wherein the reaction mixture (R_{M}) comprises at least 0.5% by weight of alkali metal alkoxide based on the total weight of the reaction mixture (R_{M}) and the molar ratio of alkali metal formate to alkali metal alkoxide in the reaction mixture (R_{M}) is greater than 1.

2. The process according to claim 1, wherein the alkali metal components of the alkali metal formate and the alkali metal alkoxide are selected independently from the group consisting of lithium, sodium, potassium, rubidium and cesium.

3. The process according to claim 1 or 2, wherein the alkali metal formate is selected from the group consisting of sodium formate and potassium formate and the alkali metal alkoxide is selected from the group consisting of sodium alkoxide and potassium alkoxide.

4. The process according to any of claims 1 to 3, wherein the alkali metal components of the alkali metal formate and of the alkali metal alkoxide are identical.

5. The process according to any of claims 1 to 4, wherein the alkali metal formate is potassium formate and the alkali metal alkoxide is potassium methoxide.

6. The process according to any of claims 1 to 5, wherein the methanol used comprises not more than 250 ppm by weight, preferably not more than 100 ppm by weight and particularly preferably not more than 50 ppm by weight, of water, in each case based on the total weight of the methanol used.

7. The process according to any of claims 1 to 6, wherein the reaction mixture (R_{M}) comprises from 0.5 to 1.5% by weight of alkali metal alkoxide and from 2.5 to 12% by weight of alkali metal formate, in each case based on the total weight of the reaction mixture (R_{M}).

8. The process according to any of claims 1 to 7, wherein the reaction mixture (R_{M}) is subjected to a further work-up comprising the following steps:
(a) separation of carbon monoxide from the reaction mixture (R_{M}) in a separation apparatus to give a gas stream (G1) comprising carbon monoxide and a liquid stream (L1) comprising methyl formate, alkali metal formate, alkali metal alkoxide and methanol,
(b) separation of the methyl formate from the liquid stream (L1) in a first distillation apparatus to give a distillate (D1) comprising methyl formate and a bottom mixture (S1) comprising alkali metal formate, alkali metal alkoxide and methanol,
(c) division of the bottom mixture (S1) into a substream (S1a) which is recirculated to the carbonylation reactor and a substream (S1b) and
(d) separation of the methanol from the substream (S1b) in a second distillation apparatus to give a distillate (D2) which comprises methanol and is recirculated to the first distillation apparatus and a bottom mixture (S2) comprising alkali metal formate and alkali metal alkoxide.

9. The process according to any of claims 1 to 8, wherein the weight ratio of substream (S1a) to substream (S1b) is greater than 50:1.

10. The process according to any of claims 1 to 9, wherein the molar ratio of alkali metal formate to alkali metal alkoxide in the reaction mixture (R_{M}) is greater than 2, particularly preferably greater than 3 and in particular greater than 5.

11. The process according to any of claims 1 to 10, wherein the reaction mixture (R_{M}) comprises
from 12 to 45% by weight of methyl formate,
from 40 to 85% by weight of methanol,
from 2.5 to 15% by weight of alkali metal formate, from 0.5 to 1.5% by weight of alkali metal alkoxide and
from 0 to 2% by weight of carbon monoxide.

12. The use of a mixture which comprises an alkali metal formate and an alkali metal alkoxide and in which the molar ratio of alkali metal formate to alkali metal alkoxide is greater than 1 as catalyst system for the preparation of methyl formate by carbonylation of methanol by means of carbon monoxide.

13. The use of a mixture according to claim 12, wherein the molar ratio of alkali metal formate to alkali metal alkoxide is greater than 2.

## Revendications

1. Procédé de fabrication de formiate de méthyle par carbonylation de méthanol avec du monoxyde de carbone dans un réacteur de carbonylation en présence d'un système catalytique qui contient un formiate alcalin et un alcoolate alcalin, pour obtenir un mélange réactionnel (R_{G}), qui contient du formiate de méthyle, du formiate alcalin, de l'alcoolate alcalin et éventuellement du méthanol non réagi et du monoxyde de carbone non réagi, et qui est soutiré du réacteur de carbonylation, le mélange réactionnel (R_{G}) contenant au moins 0,5 % en poids d'alcoolate alcalin, par rapport au poids total du mélange réactionnel (R_{G}), et le rapport molaire entre le formiate alcalin et l'alcoolate alcalin dans le mélange réactionnel (R_{G}) étant supérieur à 1.

2. Procédé selon la revendication 1, dans lequel les composants métal alcalin du formiate alcalin et de l'alcoolate alcalin sont choisis indépendamment les uns des autres dans le groupe constitué par le lithium, le sodium, le potassium, le rubidium et le césium.

3. Procédé selon la revendication 1 ou 2, dans lequel le formiate alcalin est choisi dans le groupe constitué par le formiate de sodium et le formiate de potassium, et l'alcoolate alcali est choisi dans le groupe constitué par l'alcoolate de sodium et l'alcoolate de potassium.

4. Procédé selon l'une quelconque des revendications 1 à 3, dans lequel les composants métal alcalin du formiate alcalin et de l'alcoolate alcalin sont identiques.

5. Procédé selon l'une quelconque des revendications 1 à 4, dans lequel le formiate alcalin est le formiate de potassium et l'alcoolate alcalin est le méthanolate de potassium.

6. Procédé selon l'une quelconque des revendications 1 à 5, **caractérisé en ce que** le méthanol utilisé contient au plus 250 ppm en poids, de préférence au plus 100 ppm en poids et de manière particulièrement préférée au plus 50 ppm en poids d'eau, à chaque fois par rapport au poids total du méthanol utilisé.

7. Procédé selon l'une quelconque des revendications 1 à 6, dans lequel le mélange réactionnel (R_{G}) contient 0,5 à 1,5 % en poids d'alcoolate alcalin et 2,5 à 12 % en poids de formiate alcalin, à chaque fois par rapport au poids total du mélange réactionnel (R_{G}).

8. Procédé selon l'une quelconque des revendications 1 à 7, dans lequel le mélange réactionnel (R_{G}) est davantage traité, comprenant les étapes suivantes :
(a) la séparation du monoxyde de carbone du mélange réactionnel (R_{G}) dans un dispositif de séparation pour obtenir un courant gazeux (G1), qui contient du monoxyde de carbone, et un courant liquide (L1), qui contient du formiate de méthyle, du formiate alcalin, de l'alcoolate alcalin et du méthanol,
(b) la séparation du formiate de méthyle du courant liquide (L1) dans un premier dispositif de distillation pour obtenir un distillat (D1), qui contient du formiate de méthyle, et un mélange de fond (S1), qui contient du formiate alcalin, de l'alcoolate alcalin et du méthanol,
(c) la division du mélange de fond (S1) en un courant partiel (S1a), qui est recyclé dans le réacteur de carbonylation, et un courant partiel (S1b), et
(d) la séparation du méthanol du courant partiel (S1b) dans un deuxième dispositif de distillation pour obtenir un distillat (D2), qui contient du méthanol et qui est recyclé dans le premier dispositif de distillation, et un mélange de fond (S2), qui contient du formiate alcalin et de l'alcoolate alcalin.

9. Procédé selon l'une quelconque des revendications 1 à 8, dans lequel le rapport en poids entre le courant partiel (S1a) et le courant partiel (S1b) est supérieur à 50 sur 1.

10. Procédé selon l'une quelconque des revendications 1 à 9, dans lequel le rapport molaire entre le formiate alcalin et l'alcoolate alcalin dans le mélange réactionnel (R_{G}) est supérieur à 2, de manière particulièrement préférée supérieur à 3 et de manière notamment préférée supérieur à 5.

11. Procédé selon l'une quelconque des revendications 1 à 10, dans lequel le mélange réactionnel (R_{G}) contient
| | |
|---|---|
| 12 à 45 % en poids | de formiate de méthyle, |
| 40 à 85 % en poids | de méthanol, |
| 2,5 à 15 % en poids | de formiate alcalin, |
| 0,5 à 1,5 % en poids | d'alcoolate alcalin, et |
| 0 à 2 % en poids | de monoxyde de carbone. |

12. Utilisation d'un mélange qui contient un formiate alcalin et un alcoolate alcalin, dans lequel le rapport molaire entre le formiate alcalin et l'alcoolate alcalin est supérieur à 1, en tant que système catalytique pour la fabrication de formiate de méthyle par carbonylation de méthanol avec du monoxyde de carbone.

13. Utilisation d'un mélange selon la revendication 12, dans laquelle le rapport molaire entre le formiate alcalin et l'alcoolate alcalin est supérieur à 2.
